# EUROPEAN PATENT APPLICATION

(11) **EP 1 535 631 A2**
(43) Date of publication of application: **01.06.2005**
(21) Application number: 04257230.5
(22) Date of filing: 22.11.2004
(51) Int. Cl.: A61L 15/32, A61L 15/42

(54) **Hemostatic wound dessings containing proteinaceous polymers**

(30) Priority: 25.11.2003 US 721836
(71) Applicant: ETHICON, INC., Somerville, NJ 08876 (US)
(72) Inventor: Looney, Dwayne Lee, Flemington New Jersey 08822 (US); Zhang, Guanghui, Belle Mead New Jersey 08502 (US)
(74) Representative: Mercer, Christopher Paul

(57) **Abstract**

The present invention is directed to a hemostatic wound dressing that utilizes a fibrous, fabric substrate made from a biocompatible polymer suitable for use in the body and containing a first surface and a second surface opposing the first surface, the fabric having flexibility, strength and porosity effective for use as a hemostat; and further having a porous, polymeric matrix distributed on the first and second surfaces and through the fabric substrate, the porous, polymeric matrix being made of a biocompatible, water-soluble or water-swellable proteinaceous polymer.

## Description

### FIELD OF THE INVENTION

The present invention relates to hemostatic wound dressings containing a fabric substrate and a porous, water-soluble or water-swellable proteinaceous polymeric matrix disposed on and through the substrate.

### BACKGROUND OF THE INVENTION

The control of bleeding is essential and critical in surgical procedures to minimize blood loss, to reduce post-surgical complications, and to shorten the duration of the surgery in the operating room. Due to its biodegradability and its bactericidal and hemostatic properties, cellulose that has been oxidized to contain carboxylic acid moieties, hereinafter referred to as carboxylic-oxidized cellulose, has long been used as a topical hemostatic wound dressing in a variety of surgical procedures, including neurosurgery, abdominal surgery, cardiovascular surgery, thoracic surgery, head and neck surgery, pelvic surgery and skin and subcutaneous tissue procedures.

Currently utilized hemostatic wound dressings include knitted or non-woven fabrics comprising carboxylic-oxidized cellulose. Currently utilized oxidized regenerated cellulose is carboxylic-oxidized cellulose comprising reactive carboxylic acid groups and which has been treated to increase homogeneity of the cellulose fiber. Examples of such hemostatic wound dressings commercially available include Surgicel® absorbable hemostat; Surgicel Nu-Knit® absorbable hemostat; and Surgicel® Fibrillar absorbable hemostat; all available from Johnson & Johnson Wound Management Worldwide, a division of Ethicon, Inc., Somerville, New Jersey, a Johnson & Johnson Company. Other examples of commercial absorbable hemostats containing carboxylic-oxidized cellulose include Oxycel® absorbable cellulose surgical dressing from Becton Dickinson and Company, Morris Plains, New Jersey. The oxidized cellulose hemostats noted above are knitted fabrics having a porous structure effective for providing hemostasis. They exhibit good tensile and compressive strength and are flexible such that a physician can effectively place the hemostat in position and maneuver the dressing during the particular procedure being performed.

While the absorbency of body fluid and the hemostatic action of such currently available carboxylic-oxidized cellulose hemostats are adequate for applications where mild to moderate bleeding is encountered, they are not known to be effective to provide and maintain hemostasis in cases of severe bleeding where a relatively high volume of blood is lost at a relatively high rate. In such instances, e.g. arterial puncture, liver resection, blunt liver trauma, blunt spleen trauma, aortic aneurysm, bleeding from patients with over-anticoagulation, or patients with coagulopathies, such as hemophilia, etc., a higher degree of hemostasis is required quickly.

In an effort to achieve enhanced hemostatic properties, blood-clotting agents, such as thrombin, fibrin and fibrinogen have been combined with other carriers or substrates for such agents, including gelatin-based carriers and collagen. Hemostatic wound dressings containing neutralized carboxylic-oxidized cellulose and hemostatic agents, such as thrombin, fibrinogen and fibrin are known. Neutralized carboxylic-oxidized cellulose is prepared by treating the carboxylic-oxidized cellulose with a water solution or alcohol solution of a basic salt of a weak organic acid to elevate the pH of the carboxylic-oxidized cellulose to between 5 and 8 by neutralizing the acid groups on the cellulose prior to addition of thrombin in order to make it thrombin-compatible. While such neutralized cellulose may be thrombin compatible, it is no longer bactericidal, as the anti-microbial activity of the carboxylic-oxidized cellulose provided by its acidic nature is lost.

Hemostatic agents such as thrombin, fibrinogen or fibrin, if not effectively bound chemically or physically to the substrate, may be rinsed away by blood at a wound site. The unbound agent may migrate into the blood stream, which is undesired. Methods of producing highly oxidized tri-carboxylic acid derivatives of cellulose as hemostatic materials, involving two-stage oxidation by successive processing with an iodine-containing compound and nitrogen oxides, has been disclosed in RU2146264 and IN159322. As disclosed in these disclosures, oxidized cellulosic materials were prepared by preliminary oxidation with metaperiodate or periodic acid to yield periodate-oxidized, dialdehyde cellulose to form the intermediate for forming carboxylic-oxidized cellulose. The dialdehyde cellulose intermediate then is further oxidized by NO₂ to yield the carboxylic-oxidized cellulose, which then is used as a hemostatic, anti-microbial and wound-healing agent.

It would be advantageous to provide hemostatic wound dressings that provide and maintain hemostasis in cases of severe bleeding and that maintain physical properties required for use as a wound dressing, including strength and flexibility necessary for placement and maneuvering in or on the body by a physician. It also would be advantageous to provide a hemostatic wound dressing that not only provides hemostasis and anti-microbial properties similar to or better than conventional carboxylic-oxidized cellulose-containing hemostatic wound dressings and that also is compatible with "acid-sensitive" species, but that does so without the risk of hemostatic agents migrating into the blood stream.

The present invention provides wound dressings that provide hemostatic and anti-microbial properties and/or that also may be compatible with "acid-sensitive" species.

### SUMMARY OF THE INVENTION

The present invention is directed to hemostatic wound dressings comprising a fabric substrate, said fabric substrate comprising a first surface and a second surface opposing said first surface, said fabric substrate comprising fibers and having flexibility, strength and porosity effective for use as a hemostat, said fabric and fibers comprising a biocompatible polymer suitable for use in the body; and a porous, polymeric matrix distributed on said first surface and said second surface and through said fabric substrate, said porous, polymeric matrix comprising a biocompatible, water-soluble or water-swellable proteinaceous polymer.

### Brief Description of the Figures

Figure 1 is an image produced by scanning electron microscopy (X75) of a cross section of a comparative wound dressing.
Figure 2 is an image produced by scanning electron microscopy (X75) of the wound-contact surface of a comparative wound dressing.
Figure 3 is an image produced by scanning electron microscopy (X75) of a cross section of a comparative wound dressing.
Figure 4 is an image produced by scanning electron microscopy (X75) of the wound-contact surface of a comparative wound dressing.
Figure 5 is an image produced by scanning electron microscopy (X75) of the top surface of a comparative wound dressing.
Figure 6 is an image produced by scanning electron microscopy (X100) of a cross-section of a wound dressing of the present invention.
Figure 7 is an image produced by scanning electron microscopy (X100) of the wound-contact surface of a wound dressing of the present invention.
Figure 8 is an image produced by scanning electron microscopy (X100) of the top surface of a wound dressing of the present invention.

### DETAILED DESCRIPTION OF THE INVENTION

We have discovered certain hemostatic wound dressings that utilize a fabric as a substrate, where the fabric substrate comprises fibers prepared from a biocompatible polymer(s), comprises a first surface, a second surface opposing the first surface, and that possesses properties suitable for use as a hemostat, e.g. strength, flexibility and porosity. A more detailed description of such fabric properties is presented herein below. The wound dressings further comprise a porous, polymeric matrix, preferably substantially homogeneously dispersed on the first and second surfaces and through the fabric substrate. The polymeric matrix comprises a water-soluble or water-swellable proteinaceous polymer. As used herein, proteinaceous polymer includes proteins or polypeptides comprising amino acids containing hydrophilic side chains; proteins or polypeptides containing peptide segments of amino acids containing hydrophilic side chains; and proteins or polypeptides containing hydrophilic surfaces in tertiary conformational structures. As used herein, polypeptide means a peptide comprising 30 or more amino acids.

Either of the first and second surfaces may be used to contact the wound. The hemostatic wound dressings of the present invention provide and maintain effective hemostasis when applied to a wound requiring hemostasis. Effective hemostasis, as used herein, is the ability to control and/or abate capillary, venous, or arteriole bleeding within an effective time, as recognized by those skilled in the art of hemostasis. Further indications of effective hemostasis may be provided by governmental regulatory standards and the like.

Fabrics utilized in conventional hemostatic wound dressings, such as Surgicel® absorbable hemostat; Surgicel Nu-Knit® absorbable hemostat; and Surgicel® Fibrillar absorbable hemostat; all available from Johnson & Johnson Wound Management Worldwide, a division of Ethicon, Inc., Somerville, New Jersey, a Johnson & Johnson Company, as well as Oxycel® absorbable cellulose surgical dressing from Becton Dickinson and Company, Morris Plains, New Jersey, all may be used in preparing wound dressings according to the present invention. In certain embodiments, wound dressings of the present invention are effective in providing and maintaining hemostasis in cases of severe bleeding. As used herein, severe bleeding is meant to include those cases of bleeding where a relatively high volume of blood is lost at a relatively high rate. Examples of severe bleeding include, without limitation, bleeding due to arterial puncture, liver resection, blunt liver trauma, blunt spleen trauma, aortic aneurysm, bleeding from patients with over-anticoagulation, or bleeding from patients with coagulopathies, such as hemophilia. Such wound dressings allow a patient to ambulate quicker than the current standard of care following, e.g. a diagnostic or interventional endovascular procedure.

In certain embodiments of the invention, the wound dressings may further include a hemostatic agent, or other biological or therapeutic compounds, moieties or species, including drugs and pharmaceutical agents as described in more detail herein below. The agents may be bound within the polymeric matrix, as well as to the fabric surfaces and/or within the fabric. The agents may be bound by chemical or physical means, provided that they are bound such that they do not migrate from the wound dressing upon contact with blood in the body. The hemostatic agent may be dispersed partially or homogenously through the fabric and/or the polymeric matrix. In some embodiments of the invention, the hemostatic agents, or other biological or therapeutic compounds, moieties or species, e.g. drugs, and pharmaceutical agents, may be "acid-sensitive", meaning that they may be degraded or denatured by, or otherwise detrimentally affected by acidic pH, such as is provided by conventional carboxylic-oxidized hemostatic wound dressings.

The fabric substrates utilized in the present invention may be woven or nonwoven, provided that the fabric possesses the physical properties necessary for use in hemostatic wound dressings. A preferred woven fabric has a dense, knitted structure that provides form and shape for the hemostatic wound dressings. Such fabrics are described in United States Patent Number 4,626,253, the contents of which is hereby incorporated by reference herein as if set forth in its entirety.

In preferred embodiments of the present invention, the absorbable hemostatic fabrics are warp knitted tricot fabrics constructed of bright rayon yarn which is subsequently oxidized to include carboxyl or aldehyde moieties in amounts effective to provide the fabrics with biodegradability and anti-microbial activity. The fabrics are characterized by having a single ply thickness of at least about 0.5 mm, a density of at least about 0.03 g/cm², air porosity of less than about 150 cm³/sec/cm², and liquid absorption capacity of at least about 3 times the dry weight of the fabric and at least about 0.1 g water per cm² of the fabric.

The knitted fabrics have good bulk without undue weight, are soft and drapable, and conform well to the configuration of the surface to which they are applied. The fabric may be cut into suitable sizes and shapes without running or fraying along the cut edge. Fabric strength after oxidation is adequate for use as a surgical hemostat.

Preferred hemostatic fabrics used in the present invention comprise oxidized cellulose and are best characterized by their physical properties of thickness, bulk, porosity and liquid absorption capacity, as recited above. Suitable fabrics having these properties may be constructed by knitting 60 denier, 18-filament bright rayon yarn on a 32-gauge machine at a knit quality of 12. A suitable tricot fabric construction is front-bar 1-0, 10-11; back-bar 2-3, 1-0. The extended shog movement imparted to the front bar results in a 188-inch runner compared to a 70-inch runner for the back guide bar, and increases the fabric bulk and density. The ratio of front to back bar runners in this particular construction is 1:2.7.

Typical physical and hemostatic properties of preferred fabrics produced as described above are noted in Table 1.

**TABLE I**

| Property | |
|---|---|
| Thickness (mm); | 0.645 |
| | |
| Density (g/cm²); | 0.052 |
| | |
| Air Porosity (cm³ /sec/cm²); | 62.8 |
| | |
| Tensile Strength⁽¹⁾(md/cd)Kg; | 1.9/4.5 |
| | |
| Elongation⁽²⁾ (%); | 23/49 |
| | |
| Absorption⁽³⁾ (g/g fabric); (g/cm² fabric); | 3.88 0.20 |
| | |

| Hemostasis⁽⁴⁾ (min) | |
|---|---|
| | |
| 1 ply; | 5.7 ± 1.0 |
| | |
| 2 ply; | 5.6 ± 1.8 |

| | |
|---|---|
| ⁽¹⁾ tensile strength determined at 2 in/min extension md/cd = machine direction/cross direction. | |
| ⁽²⁾ Elongation, machine direction/cross direction. | |
| ⁽³⁾ Absorption based on weight of water absorbed by fabric. | |
| ⁽⁴⁾ Hemostasis evaluation on incised porcine splenic wounds, time to stop bleeding. | |

The tricot fabrics utilized in the present invention may be constructed from bright rayon yarns of from about 40 to 80 total denier. Each yarn may contain from 10 to 25 individual filaments, although each individual filament preferably is less than 5 denier to avoid extended absorption times. The high bulk and fabric density are obtained by knitting at 28 gauge or finer, preferably at 32 gauge, with a fabric quality of about 10 or 12 (40 to 48 courses per inch). A long guide bar shog movement of at least 6 needle spaces, and preferably 8 to 12 spaces, further increases fabric thickness and density.

Other warp knit tricot fabric constructions which produce equivalent physical properties may, of course, be utilized in the manufacture of the improved hemostatic fabrics and wound dressings of the present invention, and such constructions will be apparent to those skilled in the art.

Polymers useful in preparing the fabric substrates in wound dressings of the present invention include, without limitation, collagen, calcium alginate, chitin, polyester, polypropylene, polysaccharides, polyacrylic acids, polymethacrylic acids, polyamines, polyimines, polyamides, polyesters, polyethers, polynucleotides, polynucleic acids, polypeptides, proteins, poly(alkylene oxide), polyalkylenes, polythioesters, polythioethers, polyvinyls, polymers comprising lipids, and mixtures thereof. Preferred fibers comprise oxidized regenerated polysaccharides, in particular oxidized regenerated cellulose.

Preferably, oxidized polysaccharides are used to prepare wound dressings of the present invention. More preferably, oxidized cellulose is used to prepare fabrics used in wound dressings of the present invention. The cellulose either may be carboxylic-oxidized cellulose, or may be aldehyde-oxidized cellulose, each as defmed and described herein. Even more preferably, oxidized regenerated cellulose is used to prepare fabric substrates used in wound dressings of the present invention. Regenerated cellulose is preferred due to its higher degree of uniformity versus cellulose that has not been regenerated. Regenerated cellulose and a detailed description of how to make regenerated oxidized cellulose is set forth in United States Patent 3,364,200 and United States Patent 5,180,398, the contents each of which is hereby incorporated by reference as if set forth in its entirety. As such, teachings concerning regenerated oxidized cellulose and methods of making same are well within the knowledge of one skilled in the art of hemostatic wound dressings.

Certain of the wound dressings of the present invention utilize fabric substrates that have been oxidized to contain carboxyl moieties in amounts effective to provide the fabrics with biodegradability and anti-microbial activity. U.S. Patent 3,364,200 discloses the preparation of carboxylic-oxidized cellulose with an oxidizing agent such as dinitrogen tetroxide in a Freon medium. U.S. Patent 5,180,398 discloses the preparation of carboxylic-oxidized cellulose with an oxidizing agent such as nitrogen dioxide in a per-fluorocarbon solvent. After oxidation by either method, the fabric is thoroughly washed with a solvent such as carbon tetrachloride, followed by aqueous solution of 50 percent isopropyl alcohol (IPA), and finally with 99% IPA. Prior to oxidation, the fabric is constructed in the desired woven or nonwoven construct suitable for use as a hemostat. Certain wound dressings according to the present invention that utilize such fabrics have been found to provide and maintain hemostasis in cases of severe bleeding.

Where the fabric substrate comprises carboxylic-oxidized cellulose, it has been found that the fabric preferably is conditioned prior to saturation with polymer solution and lyophilization in order to provide homogenous distribution of the polymer solution on and through the fabric substrate. Conditioning of the fabric can be achieved by storing the fabric at room temperature under ambient conditions for at least 6 month, or conditioning of the fabric can be accelerated. Preferably, the fabric is exposed to conditions of about 4°C to about 90°C, at a relative humidity of from about 5% to about 90%, for a time of from about 1 hour to 48 months. More preferably, the fabric is exposed to conditions of about 4°C to about 60°C, at a relative humidity of from about 30% to about 90%, for a time of from about 72 hours to 48 months. Even more preferably, the fabric is exposed to conditions of about 18°C to about 50°C, at a relative humidity of from about 60% to about 80%, for a time of from about 72 hours to about 840 hours. Most preferably, the fabric is conditioned at a temperature of about 50°C, at a relative humidity of about 70%, for a time of about 168 hours. The fabric may be placed horizontally in a conditioned environment, taking care to provide spacing between the fabric substrates to allow proper conditioning. The fabric also may be suspended vertically to allow conditioning.

As result of the conditioning of the carboxylic-oxidized cellulose fabric substrate, the fabric substrate will comprise at least about 3 weight percent of water-soluble molecules, preferably from about 3 to about 30 weight percent, more preferably from about 8 to about 20 weight percent, even more preferably from about 9 to about 12 weight percent, and most preferably about 10 weight percent. In general, the water-soluble molecules are acid-substituted oligosaccharides containing approximately 5 or fewer saccharide rings. It has been found that the hemostatic efficacy of the wound dressing containing such carboxylic-oxidized cellulose fabric substrates, including the occurrence of re-bleeding of a wound for which hemostasis initially has been achieved, is improved when the contents of the water-soluble molecules reach about 8%, preferably about 10%, based on the weight of the fabric substrate.

Fabric substrates used in the present invention also will comprise from about 3 to about 20 weight percent of water, preferably from about 7 to about 13 weight percent, and more preferably from about 9 to about 12 weight percent water.

Similar levels of moisture and water-soluble molecules in the carboxylic-oxidized cellulose fabric substrate also may be achieved by other means. For example, sterilization of the fabric by known techniques, such as gamma or e-beam irradiation, may provide similar content of water and/or water-soluble molecules. In addition, water-soluble molecules such as oligosaccharides could be added to the fabric prior to distribution of the porous, polymeric matrix on and through the fabric. Once having the benefit of this disclosure, those skilled in the art may readily ascertain other methods for providing such fabrics with moisture and/or water-soluble molecules.

Wound dressings of the present invention that are compatible with acid-sensitive species comprise fabric substrates prepared from a biocompatible, aldehyde-oxidized polysaccharide. In such wound dressings, the polysaccharide preferably will contain an amount of aldehyde moieties effective to render the modified polysaccharide biodegradable, meaning that the polysaccharide is degradable by the body into components that either are resorbable by the body, or that can be passed readily by the body. More particularly, the biodegraded components do not elicit permanent chronic foreign body reaction when they are absorbed by the body, such that no permanent trace or residual of the component is retained at the implantation site.

Aldehyde-oxidized polysaccharides used in the present invention may include, without limitation, cellulose, cellulose derivatives, e.g. alkyl cellulose, for instance methyl cellulose, hydroxyalkyl cellulose, alkylhydroxyalkyl cellulose, cellulose sulfate, salts of carboxymethyl cellulose, carboxymethyl cellulose and carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above, each of which has been oxidized to included anti-microbial effective amounts of aldehyde moieties.

In preferred embodiments utilizing aldehyde-oxidized polysaccharides, the polysaccharide is oxidized as described herein to assure that the aldehyde-oxidized polysaccharide is biodegradable. Such biodegradable, aldehyde-oxidized polysaccharides may be represented by Structure I below. 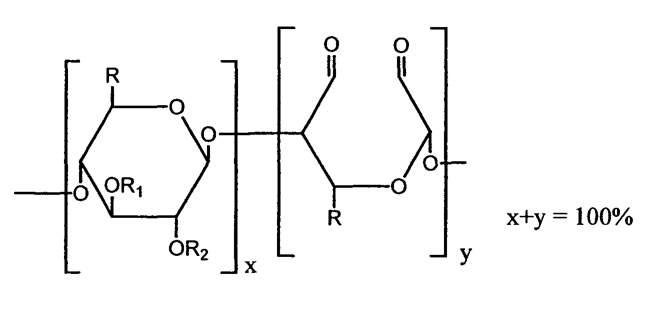 where x and y represent mole percent, x plus y equals 100 percent, x is from about 95 to about 5, y is from about 5 to about 95; and R may be CH₂OR₃, COOR₄, sulphonic acid, or phosphonic acid; R₃ and R₄ may be H, alkyl, aryl, alkoxy or aryloxy, and R₁ and R₂ may be H, alkyl, aryl, alkoxy, aryloxy, sulphonyl or phosphoryl.

In certain embodiments of the present invention, the biocompatible, biodegradable hemostatic wound dressing comprises a fabric substrate prepared from a biocompatible, biodegradable, aldehyde-oxidized regenerated cellulose. In particular, preferred aldehyde-oxidized regenerated cellulose is one comprising repeating units of Structure II: 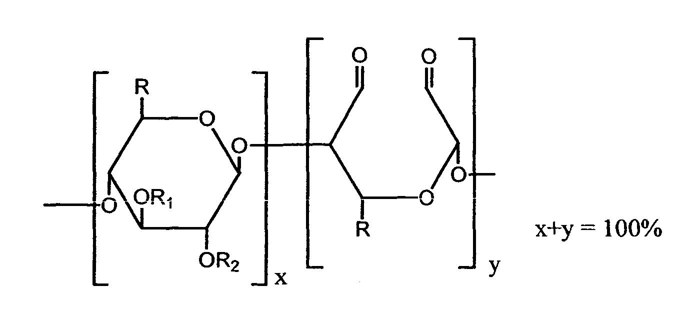 where x and y represent mole percent, x plus y equals 100 percent, x is from about 95 to about 5, y is from about 5 to about 95; and R is CH₂OH, R₁ and R₂ are H.

In other embodiments of the invention utilizing aldehyde-oxidized regenerated polysaccharides, the aldehyde-oxidized regenerated polysaccharide, e.g. cellulose, is essentially free of functional or reactive moieties other than aldehyde moieties. By essentially free, it is meant that the polysaccharide does not contain such functional or reactive moieties in amounts effective to alter the properties of the aldehyde-oxidized polysaccharide, or to provide the fabric comprising the polysaccharide with a pH of less than about 4.5, more preferably less than about 5, or greater than about 9, preferably about 9.5. Such moieties include, without limitation, carboxylic acid moieties typically present in wound dressings made from carboxyl-oxidized cellulose. Excess levels of carboxylic acid moieties will lower the pH of the fabrics and dressings so that they are not compatible for use with those acid-sensitive species that may be degraded or denatured by such a low pH, e.g. thrombin. Other moieties essentially excluded include, without limitation, sulfonyl or phosphonyl moieties.

As noted above, wound dressings of the present invention comprise a porous, polymeric matrix dispersed on the first and second surfaces and through the fabric substrate. Preferably, the matrix is dispersed substantially homogenously so as to provide the desired hemostatic properties to the wound dressing. The polymer used to prepare the porous, polymeric matrix in wound dressings of the present invention is a biocompatible, water-soluble, or water-swellable proteinaceous polymer. The water-soluble or water-swellable proteinaceous polymer rapidly absorbs blood or other body fluids and forms a tacky or sticky gel adhered to tissue when placed in contact therewith. The fluid-absorbing proteinaceous polymer, when in a dry or concentrated state, interacts with body fluid through a hydration process. Once applied in a bleeding site, the proteinaceous polymer interacts with the water component in the blood via the hydration process. The hydration force provides an adhesive interaction that aids the hemostat adhere to the bleeding site. The adhesion creates a sealing layer between the hemostat and the bleeding site to stop the blood flow.

Preferred proteinaceous polymers used to fabricate the matrices include water-swellable polypeptides, proteins or protein derivatives that are naturally occurring, recombinant or synthetic. Such proteinaceous polymers include, without limitation, albumins, algal proteins, apoproteins, blood proteins, egg proteins, lectins, lipoproteins, metalloproteins, polyproteins, collagen, elastin, fibronectins, laminin, tenascin, vitronectin, fibroin, gelatin, keratin, reticulin, poly(alpha-amino acid), poly(beta-amino acid), poly(gamma-amino acid), polyimino acid, polypeptide and derivatives of any of the above. More preferably, the water-swellable proteinaceous polymer comprises substantially non-cross-linked collagen. Non-cross-linked collagen, as used herein, is meant to include collagen where triple helices are not bonded by inter-chain chemical linkage, resulting in the collagen being more water-swellable, more adherent to tissue, more biocompatible and more penetrating to fabric substrate. The composite hemostat of the present invention remains flexible, conforms to a bleeding site and retains good tensile and compressive strength to withstand handling during application. The hemostat can be cut into different sizes and shapes to fit the surgical needs. It can be rolled up or packed into irregular anatomic areas. The fabric in a preferred embodiment capable of providing and maintaining hemostasis in cases of severe bleeding is a knitted carboxylic-oxidized regenerated cellulose, such as the fabric used to manufacture Surgicel Nu-Knit® absorbable hemostat available from Ethicon, Inc., Somerville, New Jersey.

As noted above, in certain embodiments of the invention, a biologics, a drug, a hemostatic agent, a pharmaceutical agent, or combinations thereof, that otherwise may be sensitive to the low pH of conventional carboxyl-oxidized cellulose-containing wound dressings, may be incorporated into wound dressings of the present invention without having to adjust pH prior to incorporation into the dressing. To fabricate such a hemostatic wound dressing, a drug or agent may be dissolved in an appropriate solvent. The fabric may then be coated with the drug solution and the solvent removed. Preferred biologics, drugs and agent include analgesics, anti-infective agents, antibiotics, adhesion preventive agents, pro-coagulants, and wound healing growth factors.

Hemostatic agents that may be used in combination with wound dressings according to the present invention to enhance hemostatic efficacy include, without limitation, procoagulant enzymes, proteins and peptides, can be naturally occurring, recombinant, or synthetic, and may be selected from the group consisting of prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin and vasopressin analogs, epinephrine, selectin, procoagulant venom, plasminogen activator inhibitor, platelet activating agents, synthetic peptides having hemostatic activity, derivatives of the above and any combination thereof. Preferred hemostatic agents used in the present invention are thrombin, fibrinogen and fibrin.

Hemostatic agents, such as thrombin, fibrin or fibrinogen, if bound to the wound dressing, can enhance the hemostatic property of aldehyde-oxidized regenerated cellulose wound dressings and reduce the risk of thrombosis caused by free hemostatic agents migrating into the blood stream. Hemostatic agents may be bound to the wound dressings either by chemical of physical means. Agents may be covalently conjugated with aldehyde groups pendant from the polysaccharide in one instance, thus chemically binding the agent to the wound dressing. Preferably, the hemostatic agents are physically bound to the wound dressing via incorporation into the polymeric matrix dispersed on and through the aldehyde-oxidized polysaccharide fabric and immobilized, i.e. bound, via lyophilization.

Such hemostatic wound dressings of the present invention comprise hemostatic agents, including but not limited to thrombin, fibrinogen or fibrin, in an amount effective to provide rapid hemostasis and maintain effective hemostasis in cases of severe bleeding. If the concentration of the hemostatic agent in the wound dressing is too low, the hemostatic agent does not provide an effective procoagulant activity to promote rapid clot formation upon contact with blood or blood plasma. A preferred concentration range of thrombin in the wound dressing is from about 0.001 to about 1 percent by weight. A more preferred concentration of thrombin in the wound dressing is from about 0.01 to about 0.1 percent by weight. A preferred concentration range of fibrinogen in the wound dressing is from about 0.1 to about 50 percent by weight. A more preferred concentration of fibrinogen in the wound dressing is from about 2.5 to about 10 by weight. A preferred concentration range of fibrin in the wound dressing is from about 0.1 to about 50 percent by weight. A more preferred concentration of fibrin in the wound dressing is from about 2.5 to about 10 by weight.

In certain embodiments, fabrics used in wound dressings of the present invention may comprise covalently conjugated there with a hemostatic agent bearing an aldehyde-reactive moiety. In such embodiments, the aldehyde moiety of aldehyde-oxidized regenerated polysaccharide can readily react with the amine groups present on the amino acid side chains or N-terminal residues of thrombin, fibrinogen or fibrin, resulting in forming a conjugate of the hemostatic agent with the aldehyde-oxidized regenerated polysaccharide covalently linked by a reversible imine bond. The imine bonded aldehyde-oxidized regenerated polysaccharide/hemostatic agent conjugate may then be further reacted with a reducing agent such as sodium borohydride or sodium cyanoborohydride to form an irreversible secondary amine linkage. In such embodiments of the invention, the hemostatic agent is dispersed at least on the surface of the fabric, and preferably at least partially through the fabric structure, bound reversibly or irreversibly to the aldehyde-oxidized polysaccharide.

Oxidation of 2, 3- vicinal hydroxyl groups in a carbohydrate with periodic acid (or any alkali metal salt thereof) forms a di-aldehyde or di-aldehyde derivatives. These aldehyde moieties (-RCH (O)) can then readily react with a primary amine moiety (-NH₂), such as are present on the amino acid side chains or N-terminal residues of proteins, resulting in an equilibrium with the reaction product, a protein and carbohydrate conjugate, covalently linked by a relatively unstable and reversible imine moiety (-N=CHR). To stabilize the linkage between the biomolecule and the substrate surface, subsequent reductive alkylation of the imine moiety is carried out using reducing agents (i.e., stabilizing agents) such as, for example, sodium borohydride, sodium cyanoborohydride, and amine boranes, to form a secondary amine (-NH-CH₂-R). The features of such hemostatic agents conjugated with the aldehyde-oxidized regenerated cellulose wound dressing can be controlled to suit a desired application by choosing the conditions to form the composite hemostat during conjugation.

In such embodiments of the present invention, the hemostatic agent, such as thrombin, fibrinogen or fibrin, is dispersed substantially homogeneously through the wound dressing fabric. In such cases, aldehyde-oxidized regenerated cellulose fabric may be immersed in the solution of thrombin, fibrinogen or fibrin to provide homogeneous distribution throughout the wound dressing.

In certain embodiments of the invention, the thrombin conjugate of aldehyde-oxidized regenerated cellulose fabric is further reacted with reducing agents such as sodium borohydride or sodium cyanoborohydride to form a secondary amine linkage. The aldehyde-oxidized regenerated cellulose fabric can be soaked with the desired amount of aqueous solution of thrombin, then reacted with aqueous solution of sodium borohydride or sodium cyanoborohydride reconstituted in phosphate buffer (PH=8) prior to lyophilization.

The reduced form of the aldehyde-oxidized regenerated cellulose-thrombin conjugate is more stable due to the nature of the secondary amine linkage. Hemostatic wound dressings of this embodiment have enhanced hemostatic properties, as well as increased stability, and can provide rapid hemostasis without causing thrombin to migrate into the blood stream and cause severe thrombosis.

In certain embodiments, fabrics used in wound dressings of the present invention comprise carboxylic-oxidized regenerated polysaccharide, the proteinaceous polymer matrix and an acid-sensitive hemostatic agent such as thrombin. While the combination of thrombin with such a substrate conventionally is avoided due to the expected denaturing of the thrombin by the acidic pH of the fabric, in such embodiments of the present invention, it is believed that a proteinaceous polymer matrix such as collagen provides a stabilizing affect to the hemostatic agents. Therefore, deactivation or denaturing of the "acid-sensitive" hemostatic agent such as thrombin can be prevented or reduced, if preparation of the wound dressing is conducted under certain controlled conditions. In such cases, carboxylic-oxidized regenerated cellulose fabric based wound dressings according to the present invention may be immersed in the solution of thrombin, fibrinogen or fibrin to provide distribution throughout the wound dressing, immediately followed by rapid lyophilization as exemplified in Example 2.

In preferred embodiments of the present invention, the hemostatic agent, such as thrombin, fibrinogen, or fibrin is constituted in an aqueous solution of a non-acidic, water-soluble or water-swellable proteinaceous polymer, as described herein above, including but not limited to albumins, algal proteins, apoproteins, blood proteins, egg proteins, lectins, lipoproteins, metalloproteins, polyproteins, collagen, elastin, fibronectins, laminin, tenascin, vitronectin, fibroin, gelatin, keratin, reticulin, poly(alpha-amino acid), poly(beta-amino acid), poly(gamma-amino acid), polyimino acid, polypeptides, and derivatives thereof. The oxidized regenerated cellulose fabric can be soaked with the desired amount of aqueous solution of hemostatic agent and the water-soluble or water-swellable proteinaceous polymer and rapidly lyophilized using known methods that retain therapeutic activity. When constructed thusly, the hemostatic agent will be substantially homogenously dispersed through the polymeric matrix formed during lyophilization.

One skilled in the art, once having the benefit of this disclosure, will be able to select the appropriate hemostatic agent, water-soluble or water-swellable polymer and solvent therefore, and levels of use of both the polymer and hemostatic agent, depending on the particular circumstances and properties required of the particular wound dressing.

One method of making the porous, polymeric matrix is to contact the fabric substrate with the appropriate amount of polymer solution, such that the dissolved polymer is disposed on the surfaces and substantially homogenously through the fabric, flash-freeze the polymer and fabric, and then remove the solvent from the frozen structure under vacuum, i.e. by lyophilization. The steps involved in the preparation of the novel porous structure comprise dissolving the appropriate polymer to be lyophilized in an appropriate solvent for the polymer to prepare a homogenous polymer solution. The fabric then is contacted with the polymer solution such that it is saturated with the polymer solution. The fabric substrate and polymer solution incorporated in the dense construct of the fabric then is subjected to a freezing and vacuum drying cycle. The freezing/drying step phase removes the solvent by sublimation, leaving a porous, polymer matrix structure disposed on and through the fabric substrate. Through this preferred lyophilization method, the wound dressing comprising a fabric substrate that comprises a matrix of the water-soluble or water-swellable polymer and having microporous and/or nanoporous structure is obtained. The lyophilization conditions are important to the novel porous structure in order to create a large matrix surface area in the hemostat with which body fluids can interact once the dressing is applied to a wound requiring hemostasis.

During the lyophilization process, several parameters and procedures are important to produce wound dressings having mechanical properties suitable for use in hemostatic wound dressings. The features of such microporous structure can be controlled to suit a desired application by choosing the appropriate conditions to form the composite hemostat during lyophilization. The type of microporous morphology developed during the lyophilization is a function of such factors, such as the solution thermodynamics, freezing rate, temperature to which it is frozen, and concentration of the solution. To maximize the surface area of the porous matrix of the present invention, a preferred method is to quickly freeze the fabric/polymer construct at lower than 0°C, preferably at about -50°C, and to remove the solvent under high vacuum. The porous matrix produced thereby provides a large fluid-absorbing capacity to the hemostatic wound dressing. When the hemostatic wound dressing comes into contact with body fluid, a very large surface area of polymer is exposed to the fluid instantly. The hydration force of the hemostat and subsequent formation of a tacky gelatinous layer helps to create an adhesive interaction between the hemostat and the bleeding site. The microporous structure of the polymeric matrix also allows blood to quickly pass through the fabric surface before the hydration takes place, thus providing an increased amount of the polymer to come in contact with the body fluids. The formation of a gelatinous sheet on oxidized cellulose upon blood contact will enhance the sealing property of the water-soluble gelatinous layer, which is critical to rapid hemostasis in cases ranging from moderate to severe bleeding.

The fabric substrate comprises the polymeric matrix in an amount effective to provide and maintain effective hemostasis, preferably in cases of severe bleeding. If the ratio of polymer to fabric is too low, the polymer does not provide an effective seal to physically block the bleeding, thus reducing the hemostatic properties. If the ratio is too high, the composite hemostat wound dressing will be too stiff or too brittle to conform to wound tissue in surgical applications, thus adversely affecting the mechanical properties necessary for handling by the physician in placement and manipulation of the dressing. Such an excessive ratio will also prevent the blood from quickly passing through the fabric surface to form the gelatinous layer on the oxidized cellulose that is critical for enhancing the sealing property. A preferred weight ratio of polymer to fabric is from about 1:99 to about 15:85. A more preferred weight ratio of polymer to fabric is from about 3:97 to about 10:90.

Wound dressings of the present invention are best exemplified in the figures prepared by scanning electron microscope. The samples were prepared by cutting 1-cm² sections of the dressings by using a razor. Micrographs of both the first surface and opposing second surface, and cross-sections were prepared and mounted on carbon stubs using carbon paint. The samples were gold-sputtered and examined by scanning electron microscopy (SEM) under high vacuum at 4KV.

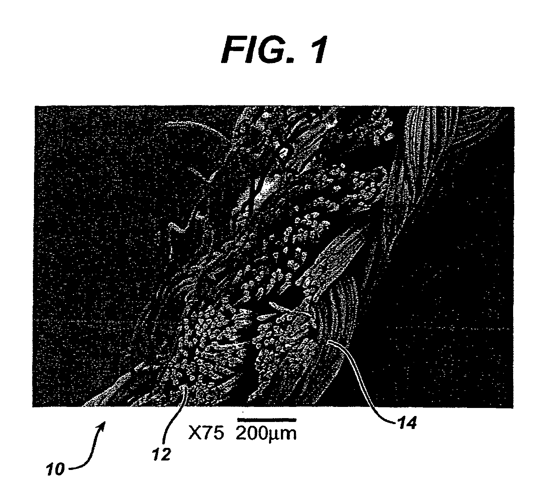

Figure 1 is a cross-section view (75X) of uncoated carboxylic-oxidized regenerated cellulose fibers 12 organized as fiber bundles 14 and knitted into fabric 10 according to preferred embodiments of the invention discussed herein above. One commercial example of such a fabric is Surgicel Nu-Knit® absorbable hemostatic wound dressing.

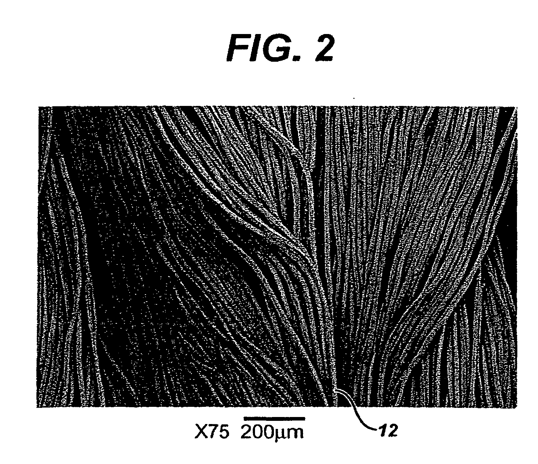

Figure 2 is a view of a first surface of the fabric of Figure 1. Individual fibers 12 are shown within a bundle.

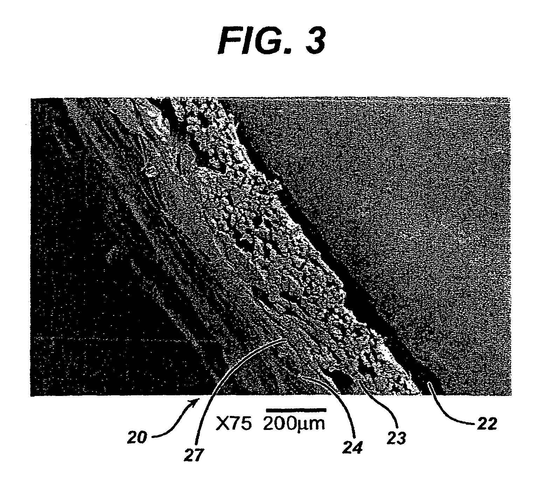

Figure 3 is a cross-section view of fabric 20 having first surface 22 and opposing surface 24 and that has been coated with a solution of sodium carboxymethyl cellulose (Na-CMC) and then air dried. Individual fibers 23 also are shown.

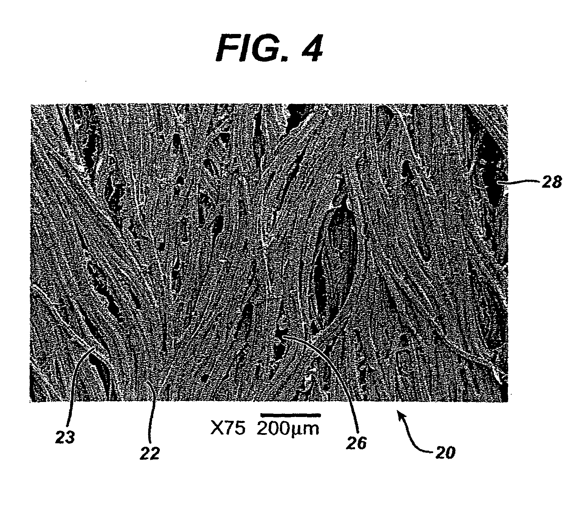

Figure 4 is a view of surface 22 of fabric 20. As observed therein, in the course of air-drying, polymer 26 agglomerates and adheres to fibers 23, in many instances adhering fibers 23 one to the other and creating large voids 28 in the hemostatic fabric through which body fluids may pass. Polymer 26 dispersed on and through fabric 20 is not in the state of a porous matrix and thus provides no hemostasis in cases of severe bleeding as described herein above due, at least in part, to a lack of sufficient porosity, e.g. surface area, to provide polymer/body fluid interaction effective to provide and maintain hemostasis in cases of severe bleeding.

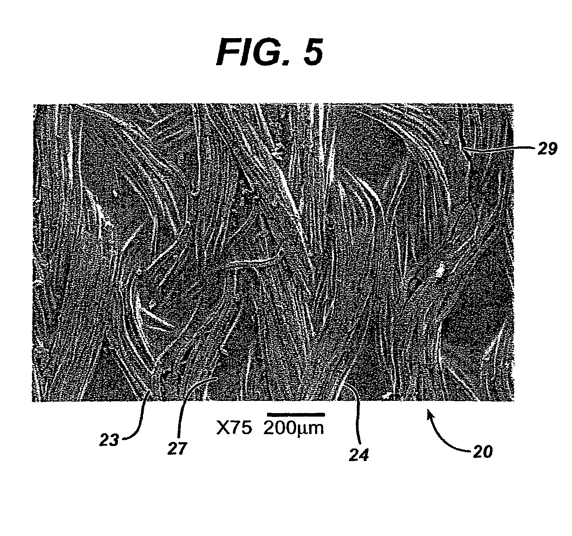

Figure 5 is a view of opposing surface 24 of fabric 20. As shown, opposing surface 24 contains a larger concentration of Na-CMC coating material as opposed to surface 22 shown in Figure 4, obscuring most of fibers 23, although the knitting pattern can still be discerned. The coating is thick enough to span across all of the fibers and generate an intact layer 27 of its own, also shown in Figure 3. This layer is brittle, as cracks 29 in the coating are observed. The coating layer thickness varied from as thin as about 3 microns in some sections to about 30-65 microns in other sections.

It is clear from Figures 3-5 that the fabrics prepared by air-drying do not contain a porous, polymeric matrix dispersed on the surfaces and there through. As such, those fabrics do not provide and maintain hemostasis in cases of severe bleeding, as shown herein. In addition, such fabrics are brittle, stiff, do not conform to wound sites, are not able to be handled by physicians, and generally are not suitable for use as wound dressings in cases of severe bleeding.

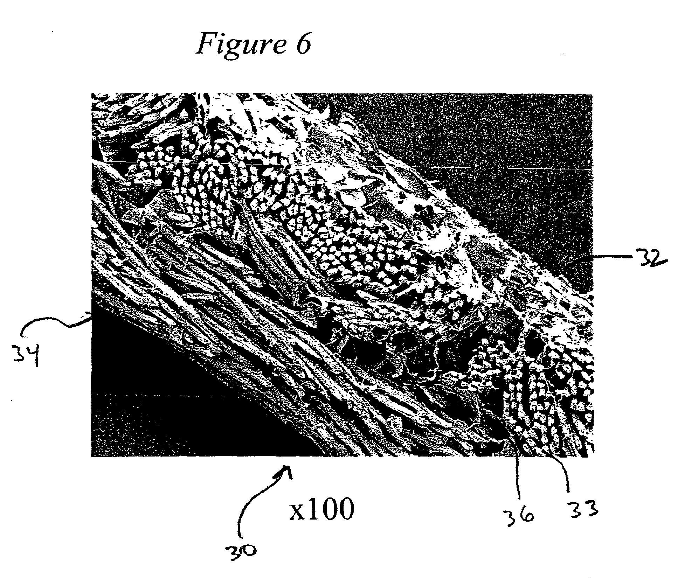

Hemostatic fabrics according to the present invention are set forth in Figure 6-8. As shown in Figures 6, a porous, polymer matrix is distributed on surfaces 32 and 34 and throughout fabric 30. Polymer 36 forms a porous matrix integrated with knitted fibers 33. The porous polymer matrix exhibits significant liquid absorption properties from capillary action in the same manner as a sponge.

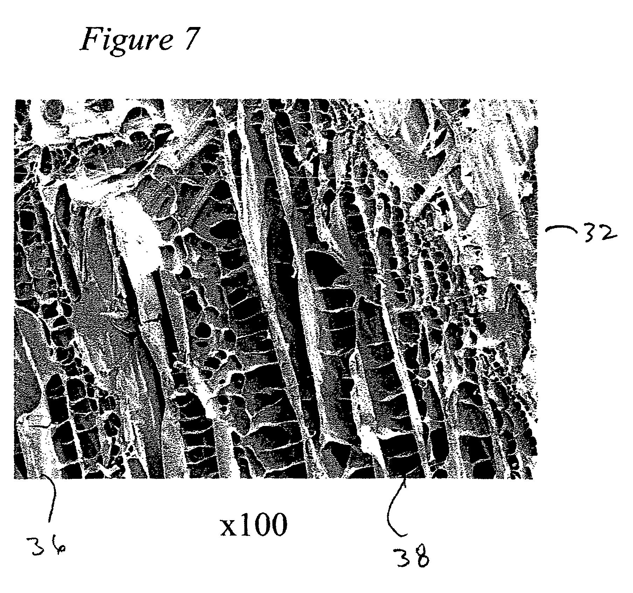

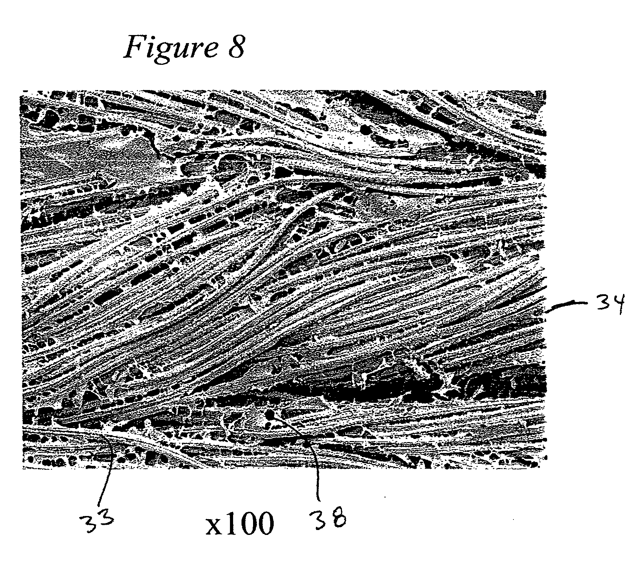

As shown in Figures 7 and 8, the matrix disposed on surfaces 32 and 34 contains countless pores, ranging from about two microns to as large as about 400 microns in diameter or greater. In preferred embodiments the pores may range from about 10 to about 35 microns. Figure 7 shows surface 32 of fabric 30. As noted, polymer 36 is present in the form of a porous matrix, thereby providing ample polymer surface area with which body fluids can interact upon contact therewith. Opposing surface 34 shown in Figure 8 also contains polymer 36 in the form of a porous matrix about fibers 33.

It is clear from Figures 6-8 that wound dressings of the present invention contain a porous polymeric matrix dispersed on the surfaces and through the fabric. Due to the porous nature of the matrix, body fluids are permitted to pass into the matrix, where ample surface area of polymer is present to interact with the body fluids. This results in faster and a higher degree of hemostasis, particularly where bleeding is occurring at a high volume and rate.

It also is clear from Figures 3-5 that comparative fabrics and wound dressings do not contain a porous, polymeric matrix, either on a surface of the dressing or dispersed throughout the fabric. As a result, the amount of polymer present to interact with body fluids is significantly reduced. In addition, due to the formation of agglomerated polymer layers during air drying, body fluids are not permitted to pass freely into the wound dressing where they can interact with and bind to the dressing. Both of these characteristics result in less hemostasis, such that wound dressings of this construct do not provide and maintain hemostasis in cases of severe bleeding. Additionally, such fabrics were found to be brittle and stiff, such that placement within and conformance to a wound site by a physician is not acceptable.

While the following examples demonstrate certain embodiments of the invention, they are not to be interpreted as limiting the scope of the invention, but rather as contributing to a complete description of the invention.

### Example 1

### Carboxylic-oxidized regenerated cellulose(CORC)/Non-cross-linked collagen patch preparation:

Four grams of collagen paste (solid collagen content 17 to 23% from bovine hide, processed by Johnson & Johnson Medical, Scotland, United Kingdom) was dispersed in 96 ml of deionized water. The mixture was treated with a homogenizer to disperse the paste and then stirred with a mechanical stirrer. After a homogeneous suspension was obtained, about 30 ml of the suspension was transferred into a stainless steel tray. A piece of Surgicel Nu-Knit® absorbable hemostat (3 in X 3 in) was placed into the tray and immersed in the collagen suspension. After one minute to allow complete wetting of the fabric, the wet fabric was transferred onto a high-density polyethylene film. The wet fabric and the film were put into a freeze-dryer and lyophilized overnight (Lyophilization cycle: -50°C /30 minutes, then -50°C/continuous vaccum/30 minutes, then -15°C /continuous vacumm/4 hours, then 0°C /continuous vacuum/4hours, then 15°C /continuous vacumm/4 hours). A very flexible patch was formed. The patch was further dried at room temperature under vacuum.

### Example 2:Carboxylic oxidized regenerated cellulose (CORC)/Non-cross-linked collagen/Thrombin patch preparation

Four grams of collagen paste (solid collagen content 17 to 23% from bovine hide, processed by Johnson & Johnson Medical, Scotland, United Kingdom) was dispersed in 96 ml of deionized water. The mixture was treated with a homogenizer to disperse the paste and then stirred with a mechanical stirrer. After a homogeneous suspension was obtained, 30,000 units of thrombin was added to 30 ml of the suspension, then transferred into a stainless steel tray. A piece of Surgicel Nu-Knit® absorbable hemostat (3 in X 3 in) was placed into the tray and immersed in the collagen/thrombin suspension. Immediately upon complete wetting of the fabric, the wet fabric was transferred onto a high-density polyethylene film. The wet fabric and the film were immediately placed into a freeze-dryer and lyophilized overnight (Lyophilization cycle: -50°C / 30 minutes, then -50°C/continuous vaccum/30 minutes, then -15°C /continuous vacumm/4 hours, then 0°C /continuous vacuum/4hours, then 15°C /continuous vacumm/4 hours). A very flexible patch was formed. The patch was further dried at room temperature under vacuum.

### Example 3

### Hemostatic performance of CORC/Collagen patch and CORC/Collagen/Thrombin patch in a porcine splenic incision model with initial tamponade for one minute

A porcine spleen incision model was used for hemostasis evaluation of different patches. The patches were cut into 2.5 cm X 1.5 cm rectangles. A linear incision of 1.5 cm with a depth of 0.3 cm was made with a surgical blade on a porcine spleen. After application of the test article, digital tamponade was applied to the incision for 1 minute. The hemostatic efficacy was then evaluated. Additional applications of digital tamponade for 30 seconds each time were used until complete hemostasis was achieved. Fabrics failing to provide hemostasis within 12 minutes were considered to be failures. Table 1 lists the results of the evaluation.

In contrast to the negative control (surgical gauze) and the conventional hemostatic material, Surgicel Nu-Knit®, the wound dressing according to the present invention provide superior hemostatic efficacy.

**Table 1**

| Materials | Time to Hemostasis (min) | Number of tamponades |
|---|---|---|
| Surgical Gauze | >12 (n = 3) | 21 |
| Surgicel Nu-Knit® | 4 (n=3) | 7 |
| CORC/Non-cross-linked collagen patch | 1: (n=3) | 1 |
| CORC/Non-cross-linked collagen/Thrombin patch | 1 (n=3) | 1 |

## Claims

1. A hemostatic wound dressing, comprising:
a fabric substrate, said fabric substrate comprising a first surface and a second surface opposing said first surface, said fabric comprising fibers and having properties effective for use as a hemostat, said fabric comprising a biocompatible polymer; and
a porous, polymeric matrix distributed on said first surface and said second surface and through said fabric substrate, said porous, polymeric matrix comprising a biocompatible, water-soluble or water-swellable proteinaceous polymer.

2. The wound dressing of claim 1 wherein said fabric comprises an oxidized polysaccharide, preferably oxidized regenerated cellulose.

3. The wound dressing of claim 2 wherein said oxidized regenerated cellulose comprises carboxylic-oxidized regenerated cellulose or aldehyde-oxidized regenerated cellulose.

4. The wound dressing of any of claims 1 to 3 wherein said water-soluble or water-swellable proteinaceous polymer is an albumin, an algal protein, an apoprotein, a blood protein, an egg protein, a lectin, a lipoproteins, a metalloprotein, a polyprotein, collagen, elastin, a fibronectin, laminin, tenascin, vitronectin, fibroin, gelatin, keratin, reticulin, poly(alpha-amino acid), poly(beta-amino acid), poly(gamma-amino acid), polyimino acid or a polypeptides.

5. The wound dressing of claim 4 wherein said proteinaceous polymer comprises non-cross-linked collagen.

6. The wound dressing of any of claims 1 to 4 wherein the weight ratio of said water-soluble or water-swellable proteinaceous polymer to said fabric is from 1:99 to 20:80.

7. The wound dressing of claim 5 wherein the weight ratio of said non-cross-linked collagen to said fabric is from 3:97 to 10:90.

8. The wound dressing of any of claims 1 to 7 further comprising a hemostatic agent.
